# EUROPEAN PATENT SPECIFICATION

(11) **EP 3 077 047 B1**
(45) Date of publication and mention of the grant of the patent: **24.04.2019**
(21) Application number: 14868582.9
(22) Date of filing: 04.12.2014
(51) Int. Cl.: A61K 31/20, A61P 3/10, A61P 3/06, A61P 3/04

(54) **ARAMCHOL SALTS**
ARAMCHOLSALZE
SELS D'ARAMCHOL

(30) Priority: 04.12.2013 US 201361911478 P
(43) Date of publication of application: 12.10.2016
(73) Proprietor: Galmed Research & Development Ltd., 6578317 Tel Aviv (IL)
(72) Inventor: BAHARAFF, Allen, 6215441 Tel Aviv (IL); ESHKAR-OREN, Idit, 6936719 Tel Aviv (IL)
(74) Representative: Korn, Richard Mervyn
(86) International application number: PCT/IL2014/051052
(87) International publication number: WO 2015/083164

(56) References cited:
- WO-A1-2010/086864
- US-A- 2 252 863
- US-A1- 2009 149 537
- US-A1- 2012 214 872

## Description

### FIELD OF THE INVENTION

The present invention relates to salts of arachidyl amido cholanoic acid (Aramchol), pharmaceutical compositions comprising same, methods for their preparation, and use thereof in medical treatment.

### BACKGROUND OF THE INVENTION

Aramchol is an amide conjugate of arachidic acid and 3-aminocholic acid, effective in reducing liver fat content as well as improving metabolic parameters associated with fatty liver disease. It belongs to a novel family of synthetic Fatty-Acid / Bile-Acid Conjugates (FABACs) and is being developed as a potentially disease modifying treatment for fatty liver disease and Non Alcoholic SteatoHepatitis (NASH).

Aramchol is chemically named 3β-arachidylamido-7α,12α-dihydroxy-5β-cholan-24-oic acid, and is represented by the following chemical structure:

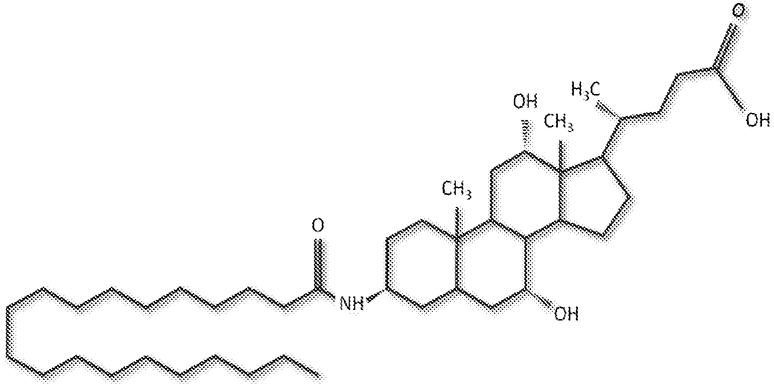

Aramchol, processes for its preparation, and use thereof are disclosed in U.S. 6,384,024; U.S. 6,395,722; U.S. 6,589,946; U.S. 7,501,403; U.S. 8,110,564; U.S. 2012/0214872; and WO 2009/060452.

There remains an unmet need for new forms of Aramchol having desirable physiochemical properties.

### SUMMARY OF THE INVENTION

The present invention provides new salts of Aramchol for example, salts with amino alcohols, amino sugars or amino acids, pharmaceutical compositions comprising said salts, methods for their preparation and use thereof in medical treatment.

The present invention is based in part on the unexpected finding of new salts of Aramchol having advantageous physicochemical properties. About 30 pharmaceutically acceptable bases were screened in an effort to prepare Aramchol salts with increased solubility. Of these, amine-based salts were found to be suitable and in particular three salts of Aramchol, namely the N-methylglucamine (meglumine), lysine and tromethamine salts have been shown to possess advantageous properties, including increased solubility, as well as increased absorption and exposure, which correlate with higher bioavailability. Thus, the Aramchol salts of the present invention are suitable for pharmaceutical use at lower doses as compared with Aramchol free acid. In addition, the new salts have improved flow properties as compared with Aramchol free acid, and therefore can be more easily processed into solid dosage formulations such as tablets or capsules.

According to a first aspect, the present invention provides a salt of 3β-arachidylamido-7α,12α-dihydroxy-5β-cholan-24-oic acid (Aramchol) with an amine. In some embodiments, the amine is selected from the group consisting of ammonia, a primary amine, a secondary amine, a tertiary amine, a quaternary ammonium compound, an amino alcohol, an amino sugar and an amino acid. Currently preferred salts are Aramchol salts with an amino alcohol, amino sugar or amino acid. Each possibility represents a separate embodiment of the present invention.

In some embodiments, the present invention provides ammonium, benzathine, trimethylglycine (betaine), ethanolamine, diethanolamine, diethylamine, arginine, lysine, choline, deanol, 2-diethylaminoethanol, N-methylglucamine (meglumine), N-ethylglucamine (eglumine) or tromethamine salt of 3β-arachidylamido-7α,12α-dihydroxy-5β-cholan-24-oic acid. Each possibility represents a separate embodiment of the present invention.

In one currently preferred embodiment, the present invention relates to 3β-arachidylamido-7α,12α-dihydroxy-5β-cholan-24-oic acid lysine salt.

In another currently preferred embodiment, the present invention relates to 3β-arachidylamido-7α,12α-dihydroxy-5β-cholan-24-oic acid tromethamine salt.

In another currently preferred embodiment, the present invention relates to 3β-arachidylamido-7α,12α-dihydroxy-5β-cholan-24-oic acid N-methylglucamine salt.

In another embodiment, the salt of 3β-arachidylamido-7α,12α-dihydroxy-5β-cholan-24-oic acid according to the present invention is in a crystalline form. In yet another embodiment, the salt of 3β-arachidylamido-7α,12α-dihydroxy-5β-cholan-24-oic acid according to the present invention is in an amorphous form.

In some embodiments, the present invention provides a method of preparing the salt of 3β-arachidylamido-7α,12α-dihydroxy-5β-cholan-24-oic acid as disclosed herein, the method comprising the steps of: (a) mixing 3β-arachidylamido-7α,12α-dihydroxy-5β-cholan-24-oic acid with an amine in the presence of a solvent; (b) optionally heating the mixture to a temperature at or below the solvent boiling point; (c) optionally cooling the mixture; and (d) isolating the thus obtained amine salt of 3β-arachidylamido-7α,12α-dihydroxy-5β-cholan-24-oic acid.

In alternative embodiments, the present invention provides a method of preparing the salt of 3β-arachidylamido-7α,12α-dihydroxy-5β-cholan-24-oic acid as disclosed herein, the method comprising the steps of: (a) mixing 3β-arachidylamido-7α,12α-dihydroxy-5β-cholan-24-oic acid with an amine in the presence of a solvent; (b) optionally heating the mixture to a temperature at or below the solvent boiling point; (c) adding an anti-solvent; (c) optionally cooling the mixture; and (d) isolating the thus obtained amine salt of 3β-arachidylamido-7α,12α-dihydroxy-5β-cholan-24-oic acid.

In some embodiments, the solvent used in the process of the invention is water. In other embodiments, the solvent is an alcohol. In particular embodiments, the solvent is methanol or ethanol. In other embodiments, the solvent is an alkyl ester such as ethyl acetate.

In some embodiments, the anti-solvent used in the process of the present invention is a ketone such as acetone or an alkyl ester such as ethyl acetate, with each possibility representing a separate embodiment of the present invention.

In some embodiments, the amine used in the process of the invention is selected from the group consisting of ammonia, a primary amine, a secondary amine, a tertiary amine, a quaternary ammonium compound, an amino alcohol, an amino sugar and an amino acid. Each possibility represents a separate embodiment of the present invention.

In certain embodiments, the ratio between the 3β-arachidylamido-7α,12α-dihydroxy-5β-cholan-24-oic acid and the amine is about 1:1. In various embodiments, the step of heating the mixture is performed to a temperature of about 50°C. In further embodiments, the step of cooling the mixture is performed to a temperature of about 20°C. In further embodiments, the step of cooling the mixture is performed to a temperature of about 5°C.

The resulting 3β-arachidylamido-7α,12α-dihydroxy-5β-cholan-24-oic acid salt resulting from the above mentioned methods may be isolated by any method known in the art, for example by evaporating the solvent so as to obtain a solid, or by forming a precipitate of the salt (e.g., by addition of an anti-solvent), and separating the precipitate from the reaction mixtures, e.g., by filtration.

In some aspects and embodiments, the present invention provides a pharmaceutical composition comprising (a) a therapeutically effective amount of a salt of 3β-arachidylamido-7α,12α-dihydroxy-5β-cholan-24-oic acid as disclosed herein; and optionally (b) at least one pharmaceutically acceptable carrier, diluent, vehicle or excipient.

In several embodiments, the pharmaceutical composition is in a form selected from the group consisting of tablets, pills, capsules, pellets, granules, powders, lozenges, sachets, cachets, patches, elixirs, suspensions, dispersions, emulsions, solutions, syrups, aerosols, ointments, soft and hard gelatin capsules, suppositories, sterile injectable solutions, and sterile packaged powders. Each possibility represents a separate embodiment of the present invention.

In other embodiments, the present invention provides a pharmaceutical composition comprising (a) a therapeutically effective amount of a salt of 3β-arachidylamido-7α,12α-dihydroxy-5β-cholan-24-oic acid as disclosed herein; and (b) at least one pharmaceutically acceptable carrier, diluent, vehicle or excipient, for use in reducing cholesterol levels in the blood or treating fatty liver, or for the treatment of Non Alcoholic SteatoHepatitis (NASH) or any disease that its treatment may benefit from modulating cholesterol or lipid balance.

In some embodiments, the pharmaceutical composition of the present invention is used for dissolving cholesterol gallstones in bile and for preventing formation of such gallstones. In other embodiments, the pharmaceutical composition of the present invention is used for treating arteriosclerosis.

In certain embodiment, the pharmaceutical composition of the present invention is used for treating a disease or disorder associated with altered glucose metabolism. In one embodiment, the disease or disorder associated with altered glucose metabolism is selected from the group consisting of hyperglycemia, diabetes, insulin resistance, and obesity. Each possibility represents a separate embodiment of the present invention.

In other embodiments, the pharmaceutical composition of the present invention is used for treating, preventing, or inhibiting progression of a brain disease characterized by amyloid plaque deposits. In one embodiment, the brain disease characterized by amyloid plaque deposits is Alzheimer's disease.

The pharmaceutical composition of the present invention can be administered via a route selected from the group consisting of oral, topical, subcutaneous, intraperitoneal, rectal, intravenous, intra-arterial, transdermal, intramuscular, and intranasal. Each possibility represents a separate embodiment of the present invention.

In some embodiments, the present invention provides a method of reducing cholesterol levels in the blood or treating fatty liver, or treating NASH, or dissolving cholesterol gallstones in bile and preventing formation of such gallstones or treating arteriosclerosis comprising administering to a subject in need thereof a pharmaceutical composition comprising (a) a therapeutically effective amount of a salt of 3β-arachidylamido-7α,12α-dihydroxy-5β-cholan-24-oic acid as disclosed herein; and (b) at least one pharmaceutically acceptable carrier, diluent, vehicle or excipient.

In certain embodiments, present invention provides a method of treating a disease or disorder associated with altered glucose metabolism comprising administering to a subject in need thereof a pharmaceutical composition comprising (a) a therapeutically effective amount of a salt of 3β-arachidylamido-7α,12α-dihydroxy-5β-cholan-24-oic acid as disclosed herein; and (b) at least one pharmaceutically acceptable carrier, diluent, vehicle or excipient. In further embodiments, the present invention provides a method of treating, preventing, or inhibiting progression of a brain disease characterized by amyloid plaque deposits comprising administering to a subject in need thereof a pharmaceutical composition comprising (a) a therapeutically effective amount of a salt of 3β-arachidylamido-7α,12α-dihydroxy-5β-cholan-24-oic acid as disclosed herein; and (b) at least one pharmaceutically acceptable carrier, diluent, vehicle or excipient.

In some embodiments, the subject is a mammal, preferably a human.

Further embodiments and the full scope of applicability of the present invention will become apparent from the detailed description given hereinafter.

### BRIEF DESCRIPTION OF THE FIGURES

**FIG. 1** illustrates a characteristic X-ray diffraction pattern of amorphous Aramchol N-methylglucamine (meglumine) salt according to the present invention.
**FIG. 2** illustrates a characteristic X-ray diffraction pattern of amorphous Aramchol lysine salt according to the present invention.
**FIG. 3** illustrates a characteristic X-ray diffraction pattern of amorphous Aramchol tromethamine salt according to the present invention.
**FIG. 4** illustrates a characteristic ¹H-NMR spectrum of Aramchol N-methylglucamine salt according to the present invention.
**FIG. 5** illustrates a characteristic ¹H-NMR spectrum of Aramchol lysine salt according to the present invention.
**FIG. 6** illustrates a characteristic ¹H-NMR spectrum of Aramchol tromethamine salt according to the present invention.
**FIG. 7** illustrates a characteristic ¹H-NMR spectrum of Aramchol free acid.
**FIG. 8** illustrates a characteristic Dynamic Vapour Sorption (DVS) spectrum of Aramchol N-methylglucamine salt according to the present invention.
**FIG. 9** AUC/dose calculated for Aramchol (free acid), N-methylglucamine, tromethamine and lysine salts. Data are arithmetic mean ± standard error.

### DETAILED DESCRIPTION OF THE INVENTION

The present invention relates to salts of Aramchol which exhibit improved physicochemical properties including increased solubility, increased absorption, and increase exposure which correlates with higher bioavailability as compared with Aramchol free acid.

According to the principles of the present invention, provided herein is a pharmaceutically acceptable salt of Aramchol in which the counter ion is based on an amine and includes ammonia, a primary amine, a secondary amine, a tertiary amine, a quaternary ammonium compound, an amino alcohol, an amino sugar or an amino acid. The amine may also be a diamine or a cyclic amine. Currently preferred salts are N-methylglucamine (meglumine), lysine or tromethamine salts. Each possibility represents a separate embodiment of the present invention.

As used herein, the term "primary amine" designates a compound of formula R^{a}NH₂ wherein R^{a} is alkyl, cycloalkyl or aryl. Examples of primary amines are lower alkylamines wherein lower alkyl means a C₁-C₄ alkyl, or arylamines. The primary amine may react with the carboxylic acid group of Aramchol to form the salt Aramchol-COO⁻ R^{a}NH₃⁺.

As used herein, the term "secondary amine" designates a compound of formula R^{a}R^{b}NH wherein each of R^{a} and R^{b} is independently alkyl, cycloalkyl or aryl. Examples of secondary amines are lower dialkylamines (R^{a}, R^{b} are each a lower alkyl), diarylamines, or akylarylamines. The secondary amine may also be a cyclic amine (e.g., morpholine, pyrrolidine, piperidine, etc.), or a diamine (e.g., benzathaine). The secondary amine may react with the carboxylic acid group of Aramchol to form the salt Aramchol-COO⁻ R^{a}R^{b}NH₂⁺.

As used herein, the term "tertiary amine" designates a compound of formula R^{a}R^{b}R^{c}N wherein each of R^{a}, R^{b} and R^{c} is independently alkyl, cycloalkyl or aryl. Examples of tertiary amines are lower trialkylamines (R^{a}, R^{b} and R^{c} are each a lower alkyl), triarylamines, or any combination of alkylarylamines. The tertiary amine may also be a cyclic amine (e.g., N-methyl pyrrolidine, N-methylpiperidine, etc.) or a diamine. The tertiary amine may react with the carboxylic acid group of Aramchol to form the salt Aramchol-COO⁻ R^{a}R^{b}R^{c}NH⁺.

As used herein, the term "quaternary ammonium compound" designates a compound of formula R^{a}R^{b}R^{c}R^{d}N⁺ X⁻ wherein each of R^{a}, R^{b}, R^{c} and R^{d} is independently alkyl, cycloalkyl or aryl and X⁻ is a counter-ion. Examples of quaternary ammonium compounds are lower tetraalkylamines (R^{a}, R^{b}, R^{c} and R^{d} are each a lower alkyl), tetraarylamines, or any combination of alkylarylamines. Specific examples of quaternary ammonium compounds which may form salts with Aramchol according to the present invention are Bu₄N⁺X⁻, choline (Me₃N⁺CH₂CH₂OH]X⁻) or trimethylglycine ((CH₃)₃N⁺CH₂CO₂HX⁻, also known as betaine), wherein X is a counter-ion, for example OH, halogen (F, Cl, Br, I) and the like. The quaternary ammonium compound may react with the carboxylic acid group of Aramchol to form the salt Aramchol-COO⁻ R^{a}R^{b}R^{c}R^{d}N⁺.

As used herein, the term "amino alcohol" or "alkanolamine", used herein interchangeably means compounds that contain both hydroxy (-OH) and amino (-NH₂, -NHR, and -N(R)₂) functional groups on an alkane backbone. Examples include but are not limited to tromethamine, ethanolamine, diethanolamine, 2-diethylaminoethanol and 2-dimethylaminoethanol.

As used herein, the term "amino sugar" or "amino sugar alcohol" means a sugar or sugar alcohol moiety in which one of the sugar hydroxyls has been replaced by an amino group. Examples of amino sugars are N-alkyl glucamines, for example N-methylglucamine (meglumine), N-ethylglucamine (eglumine), N-propylglucamine, N-butylglucamine and the like.

Thus, in some exemplary embodiments, the present invention provides salts of Aramchol with suitable organic amines such as, but not limited to, unsubstituted or substituted lower alkylamines, diamines, saturated cyclic amines, and quaternary ammonium compounds. Each possibility represents a separate embodiment of the present invention. Particular examples include, but are not limited to, methylamine, dimethylamine, trimethylamine, triethylamine, ethylamine, diethylamine, ethylenediamine, ethanolamine, diethanolamine, triethanolamine, tromethamine (TRIS), 1-amino-2-propanol, 3-amino-1-propanol, hexamethylenetetramine, deanol, 2-diethylaminoethanol, N-methylglucamine (meglumine), N-ethylglucamine (eglumine), piperidine, piperazine, pyrrolidine, morpholine, benzathine, trimethylglycine (betaine), choline and the like. Each possibility represents a separate embodiment of the present invention.

In some aspects and embodiments, the present invention provides the N-methylglucamine (meglumine) salt of Aramchol. In one embodiment, the N-methylglucamine salt of Aramchol is amorphous.

In further aspects and embodiments, the present invention provides the tromethamine (TRIS) salt of Aramchol. In one embodiment, the tromethamine salt of Aramchol is amorphous.

In further aspects and embodiments, the present invention provides the ammonium salt of Aramchol. In one embodiment, the ammonium salt of Aramchol is crystalline. In another embodiment, the ammonium salt of Aramchol is characterized by a DSC-TGA thermogram having a peak at about 76°C with an onset at about 60°C and a peak at about 117°C with an onset at about 114°C. In specific embodiments, the peak at about 76°C is accompanied by weight loss of about 2%. In yet another embodiment, the ammonium salt of Aramchol is characterized by a DSC-TGA thermogram having a peak at about 57°C with an onset at about 55°C. In particular embodiments, the peak at about 57°C is accompanied by weight loss of about 5%.

In other aspects and embodiments, the present invention provides the benzathine salt of Aramchol. In one embodiment, the benzathine salt of Aramchol is amorphous.

In further aspects and embodiments, the present invention provides the trimethylglycine (betaine) salt of Aramchol. In one embodiment, the trimethylglycine (betaine) salt of Aramchol is amorphous.

In yet other aspects and embodiments, the present invention provides the ethanolamine salt of Aramchol. In one embodiment, the ethanolamine salt of Aramchol is amorphous. In another embodiment, the ethanolamine salt of Aramchol is crystalline. In specific embodiments, the crystalline ethanolamine salt of Aramchol is characterized by a DSC-TGA thermogram having a peak at about 50°C with an onset at about 45°C, a peak at about 72°C with an onset at about 63°C, a peak at about 86°C with an onset at about 80°C, and a peak at about 122°C with an onset at about 105°C. In particular embodiments, the peaks are characterized by a continuous weight loss of about 25%.

In certain aspects and embodiments, the present invention provides the diethanolamine salt of Aramchol. In one embodiment, the diethanolamine salt of Aramchol is amorphous.

In additional aspects and embodiments, the present invention provides the diethylamine salt of Aramchol. In one embodiment, the diethylamine salt of Aramchol is amorphous.

In other aspects and embodiments, the present invention provides the choline salt of Aramchol. In one embodiment, the choline salt of Aramchol is amorphous.

In yet other aspects and embodiments, the present invention provides the deanol salt of Aramchol. In one embodiment, the deanol salt of Aramchol is amorphous.

In several aspects and embodiments, the present invention provides the 2-diethylaminoethanol salt of Aramchol. In one embodiment, the 2-diethylaminoethanol salt of Aramchol is amorphous.

In some aspects and embodiments, the present invention provides the amino acids salts of Aramchol including, but not limited to basic amino acids such as lysine, arginine, histidine, and ornithine. Each possibility represents a separate embodiment of the present invention. The amino acids, according to the principles of the present invention, may be D-amino acids, L-amino acids, or racemic derivatives of amino acids. In one embodiment, the present invention provides the arginine salt of Aramchol. In another embodiment, the present invention provides the lysine salt of Aramchol. In some embodiments, the amino acids salts of Aramchol are other than the glycine and taurine salts of Aramchol. In certain embodiments, the amino acids salts of Aramchol are amorphous. A currently preferred amino acid salt of Aramchol is the lysine salt. In some embodiments, the lysine salt is amorphous.

It is understood that the pharmaceutically acceptable salts of the present invention, when isolated in solid or crystalline form, also include hydrates or water molecules entrapped therein.

The present invention further provides methods for the preparation of Aramchol salts of the present invention. The methods utilize Aramchol free acid which is prepared by any method known in the art, including, for example, the methods described in U.S. 6,384,024; U.S. 6,395,722; U.S. 6,589,946; U.S. 7,501,403; U.S. 8,110,564; U.S. 2012/0214872; and WO 2009/060452. The contents of the aforementioned references are incorporated by reference herein. It is to be understood that the conjugation between the fatty acid radical and the bile acid in Aramchol can be in the α or the β configuration. Each possibility represents a separate embodiment of the present invention. According to one embodiment, the Aramchol free acid is mixed with the corresponding base of the salt to be formed, typically in a 1:1 ratio in the presence of a suitable solvent. The mixture is then optionally heated to temperatures which are above room temperatures but below the solvent boiling point or at the solvent boiling point (i.e., reflux). Typically the mixture is heated to about 50°C. The mixture is optionally cooled to temperatures, typically below room temperatures (e.g. 5°C). The thus obtained salt of the present invention is then isolated as is known in the art, for example by evaporation of the solvent, crystallization, precipitation with anti-solvent and the like. Each possibility represents a separate embodiment of the present invention.

In one particular embodiment, the Aramchol free acid is mixed with the corresponding base of the salt to be formed, typically in a 1:1 ratio in the presence of a suitable solvent. The mixture is then optionally heated as described above. An anti-solvent is then added and the mixture is optionally cooled as described above, so as to form a precipitate of the Aramchol salt.

Additional methods for the preparation of the Aramchol salts of the present invention include, for example, precipitation by cooling under vacuum, sublimation, saponification, growth from a melt, solid state transformation from another phase, precipitation from a supercritical fluid, and jet spraying. Each possibility represents a separate embodiment of the present invention. Techniques for precipitation from a solvent or solvent mixture include, for example, evaporation of the solvent, decreasing the temperature of the solvent mixture, freeze-drying the solvent mixture, and addition of anti-solvents (counter-solvents) to the solvent mixture. Each possibility represents a separate embodiment of the present invention.

The Aramchol salts of the present invention can be amorphous or crystalline in any polymorphic form.

Suitable solvents for preparing the salts of the present invention include polar and non-polar solvents. The choice of solvent or solvents is typically dependent upon one or more factors, including the solubility of the compound in such solvent and vapor pressure of the solvent. Combinations of solvents may be employed according to the principles of the present invention. Suitable solvents include, but are not limited to, polar aprotic solvents, polar protic solvents, and mixtures thereof. Each possibility represents a separate embodiment of the present invention. Particular examples of suitable polar protic solvents include, but are not limited to, water and alcohols such as methanol (MeOH), ethanol (EtOH), 1-butanol, and isopropanol (IPA), as well as organic esters and ketones such as ethyl acetate (EtOAc) or acetone. Each possibility represents a separate embodiment of the present invention. In one embodiment, the solvent is water. In another embodiment, the solvent is ethanol. In another embodiment, the solvent is ethyl acetate.

The anti-solvent may be any of the solvents described above, with a currently preferred anti-solvent being acetone or ethyl acetate.

The novel salts of the present invention are useful as pharmaceuticals for medical treatment. The present invention thus provides pharmaceutical compositions comprising any of the Aramchol salts disclosed herein and at least one pharmaceutically acceptable carrier, diluent, vehicle or excipient. The salts of the present invention can be safely administered orally or non-orally. Routes of administration include, but are not limited to, oral, topical, subcutaneous, intraperitoneal, rectal, intravenous, intra-arterial, transdermal, intramuscular, topical, and intranasal. Each possibility represents a separate embodiment of the present invention. Additional routes of administration include, but are not limited to, mucosal, nasal, parenteral, gastrointestinal, intraspinal, intrauterine, intraocular, intradermal, intracranial, intratracheal, intravaginal, intracerebroventricular, intracerebral, ophthalmic, buccal, epidural and sublingual. Each possibility represents a separate embodiment of the present invention. Typically, the Aramchol salts of the present invention are administered orally.

The pharmaceutical compositions can be formulated as tablets (including e.g. film-coated tablets), powders, granules, capsules (including soft capsules), orally disintegrating tablets, pills, pellets, lozenges, sachets, cachets, patches, elixirs, suspensions, dispersions, emulsions, solutions, syrups, aerosols, ointments, soft and hard gelatin capsules, suppositories, sterile injectable solutions, sterile packaged powders, and sustained-release preparations as is well known in the art. Each possibility represents a separate embodiment of the present invention.

Pharmacologically acceptable carriers, diluents, vehicles or excipients that may be used in the context of the present invention include, but are not limited to, surfactants, lubricants, binders, fillers, compression aids, disintegrants, water-soluble polymers, inorganic salts, preservatives, antioxidants, coloring agents, sweetening agents, souring agents, bubbling agents and flavorings. Each possibility represents a separate embodiment of the present invention.

Specific non-limiting examples of suitable carriers, diluents, vehicles or excipients include e.g. lactose, D-mannitol, starch, cornstarch, crystalline cellulose, light silicic anhydride and titanium oxide. Each possibility represents a separate embodiment of the present invention. Suitable surfactants include e.g. lecithin and phosphatidylcholine. Each possibility represents a separate embodiment of the present invention. Suitable lubricants include e.g. magnesium stearate, sucrose fatty acid esters, polyethylene glycol, talc and stearic acid. Each possibility represents a separate embodiment of the present invention. Suitable binders include e.g. hydroxypropyl cellulose, hydroxypropylmethyl cellulose, crystalline cellulose, α-starch, polyvinylpyrrolidone, gum arabic powder, gelatin, pullulan and low-substitutional hydroxypropyl cellulose. Each possibility represents a separate embodiment of the present invention. Suitable disintegrants include e.g. crosslinked povidone (any crosslinked 1-ethenyl-2-pyrrolidinone homopolymer including polyvinylpyrrolidone (PVPP) and 1-vinyl-2-pyrrolidinone homopolymer), crosslinked carmellose sodium, carmellose calcium, carboxymethyl starch sodium, low-substituted hydroxypropyl cellulose, cornstarch and the like. Each possibility represents a separate embodiment of the present invention. Suitable water-soluble polymers include e.g. cellulose derivatives such as hydroxypropyl cellulose, polyvinylpyrrolidone, hydroxypropylmethyl cellulose, methyl cellulose and carboxymethyl cellulose sodium, sodium polyacrylate, polyvinyl alcohol, sodium alginate, guar gum, and the like. Each possibility represents a separate embodiment of the present invention. Suitable inorganic salts include e.g. basic inorganic salts of sodium, potassium, magnesium and/or calcium. Each possibility represents a separate embodiment of the present invention. Particular embodiments include the basic inorganic salts of magnesium and/or calcium. Basic inorganic salts of sodium include, for example, sodium carbonate, sodium hydrogen carbonate, disodiumhydrogenphosphate, and the like. Each possibility represents a separate embodiment of the present invention. Basic inorganic salts of potassium include, for example, potassium carbonate, potassium hydrogen carbonate, and the like. Each possibility represents a separate embodiment of the present invention. Basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite, aluminahydroxidemagnesium, and the like. Each possibility represents a separate embodiment of the present invention. Basic inorganic salts of calcium include, for example, precipitated calcium carbonate, calcium hydroxide, and the like. Each possibility represents a separate embodiment of the present invention.

Suitable preservatives include e.g. sodium benzoate, benzoic acid, and sorbic acid. Each possibility represents a separate embodiment of the present invention. Suitable antioxidants include e.g. sulfites, ascorbic acid and α-tocopherol. Each possibility represents a separate embodiment of the present invention. Suitable coloring agents include e.g. food colors such as Food Color Yellow No. 5, Food Color Red No. 2 and Food Color Blue No. 2, and the like. Each possibility represents a separate embodiment of the present invention. Suitable sweetening agents include e.g. dipotassium glycyrrhetinate, aspartame, stevia and thaumatin. Each possibility represents a separate embodiment of the present invention. Suitable souring agents include e.g. citric acid (citric anhydride), tartaric acid and malic acid. Each possibility represents a separate embodiment of the present invention. Suitable bubbling agents include e.g. sodium bicarbonate. Suitable flavorings include synthetic substances or naturally occurring substances, including e.g. lemon, lime, orange, menthol and strawberry. Each possibility represents a separate embodiment of the present invention.

In some embodiments, the present invention provides a pharmaceutical composition comprising as an active ingredient a single Aramchol salt of the present invention and at least one pharmaceutically acceptable carrier, diluent, vehicle or excipient. In other embodiments, the present invention provides a pharmaceutical composition comprising as an active ingredient a plurality of Aramchol salts of the present invention and at least one pharmaceutically acceptable carrier, diluent, vehicle or excipient.

The Aramchol salts of the present invention are particularly suitable for oral administration in the form of tablets, capsules, pills, dragees, powders, granules and the like. Each possibility represents a separate embodiment of the present invention. A tablet may be made by compression or molding, optionally with one or more excipients as is known in the art. Specifically, molded tablets may be made by molding in a suitable machine a mixture of the powdered active ingredient moistened with an inert liquid diluent.

The tablets and other solid dosage forms of the pharmaceutical compositions described herein may optionally be scored or prepared with coatings and shells, such as enteric coatings and other coatings well known in the art. They may also be formulated so as to provide slow or controlled release of the active ingredient therein using, for example, hydroxypropylmethyl cellulose in varying proportions to provide the desired release profile, other polymer matrices and the like. The active ingredient can also be in micro-encapsulated form, if appropriate, with one or more of the above-described excipients.

The present invention provides a method of reducing cholesterol levels in the blood or treating fatty liver comprising administering to a subject in need thereof a therapeutically effective amount of a composition comprising any one of the Aramchol salts of the present invention. The present invention provides a method of treating fatty liver disease and non-alcoholic SteatoHepatitis (NASH) comprising administering to a subject in need thereof a therapeutically effective amount of a composition comprising any one of the Aramchol salts of the present invention. The present invention further provides a method of dissolving cholesterol gallstones in bile and for preventing formation of such gallstones comprising administering to a subject in need thereof a therapeutically effective amount of a composition comprising any one of the Aramchol salts of the present invention. In other embodiments, the present invention provides a method of treating arteriosclerosis comprising administering to a subject in need thereof a therapeutically effective amount of a composition comprising any one of the Aramchol salts of the present invention. The present invention also provides a method of treating a disease or disorder associated with altered glucose metabolism, particularly hyperglycemia, diabetes, insulin resistance and obesity, comprising administering to a subject in need thereof a therapeutically effective amount of a composition comprising any one of the Aramchol salts of the present invention. The present invention further provides a method of treating, preventing, or inhibiting progression of a brain disease characterized by amyloid plaque deposits, particularly Alzheimer's disease, comprising administering to a subject in need thereof a therapeutically effective amount of a composition comprising any one of the Aramchol salts of the present invention.

A "therapeutically effective amount" as used herein refers to an amount of an agent which is effective, upon single or multiple dose administration to the subject in providing a therapeutic benefit to the subject. In additional embodiments, the Aramchol salts of the present invention are used for the preparation of a medicament for treating the aforementioned diseases or disorders.

The following examples are presented in order to more fully illustrate certain embodiments of the invention. They should in no way, however, be construed as limiting the broad scope of the invention. One skilled in the art can readily devise many variations and modifications of the principles disclosed herein without departing from the scope of the invention.

### Example 1 - Synthesis of Aramchol Salts:

The Aramchol salts of the present invention were prepared according to the following procedure: Aramchol free acid was mixed with the corresponding base in a ratio of 1:1 in water or ethanol. The mixture was heated to 50°C at a rate of 1°C/min. The mixture was kept at 50°C for 2 hours, and cooled at a rate of 0.1°C/min to 20°C. In cases where the salts did not precipitate out after cooling, the crude reaction mixtures were maintained for 3 days and the purity was measured by HPLC. The Aramchol salts which provided a clear solution showed no additional impurities on HPLC. The results are summarized in Table 1.

The following Aramchol salts were found to be soluble (> 50 mg/ml at 50°C) in water: L-arginine salt, choline salt, N-methylglucamine salt, diethylamine salt, 2-diethylamino-ethanol salt, deanol salt, ethanolamine salt, and diethanolamine salt. The following Aramchol salts were found to be soluble (> 50 mg/ml at 50°C) in ethanol at 50°C: L-arginine salt, choline salt, trimethylglycine (betaine) salt, diethylamine salt, benzathine salt, 2-diethylamino-ethanol salt, deanol salt, tromethamine salt, and diethanolamine salt. No salts were obtained using glycine or taurine.

Using water as a solvent, the following Aramchol salts precipitated as amorphous material: L-arginine salt, L-lysine salt, choline salt, N-methylglucamine salt, diethylamine salt, benzathine salt, 2-diethylamino-ethanol salt, deanol salt, ethanolamine salt, and diethanolamine salt. A crystalline ammonium salt of Aramchol was obtained from water (Form I). The form was characterized by thermal analysis. The DSC profile showed a first peak at 76.32°C with an onset at 60.07°C (ΔE= -29.33J/g) and a second peak at 117.12°C with an onset at 114.08°C (ΔE= - 67.16J/g). The weight loss during the first peak was 2.05%.

**Table 1.**

| **Base** | **Dissolved (50 mg/ml) at 50°C** | **XRPD** | **salt remains in solution after cooling to 20°C** | **Stability in water (HPLC) after 3 days** |
|---|---|---|---|---|
| L-Arginine | Yes | n.a. | no | - |
| L-Lysine | No | Starting material | - | - |
| Choline | Yes | n.a. | yes | good |
| Ammonia | No | crystalline | no | - |
| N-methylglucamine | Yes | n.a. | no | - |
| Trimethylglycine (betaine) | No | Starting material | - | - |
| Diethylamine | Yes | n.a. | no | - |
| Benzathine | No | Amorphous | - | - |
| 2-diethylamino-ethanol | Yes | n.a. | yes | good |
| Deanol | Yes | n.a. | yes | good |
| Tromethamine | No | Starting material | - | - |
| Ethanolamine | Yes | n.a. | no | - |
| Diethanolamine | Yes | n.a. | yes | good |

| | | | | |
|---|---|---|---|---|
| n.a.= not available | | | | |

Using ethanol as a solvent, the following Aramchol salts precipitated as amorphous material: L-arginine salt, choline salt, trimethylglycine (betaine) salt, diethylamine salt, benzathine salt, 2-diethylamino-ethanol salt, deanol salt, tromethamine salt, and diethanolamine salt. A crystalline ammonium salt of Aramchol was obtained from ethanol. The form was characterized by thermal analysis. The DSC profile showed a peak at 56.57°C with an onset at 55.37°C (ΔE= -45.57J/g). The weight loss during the peak was 5.44%. A crystalline ethanolamine salt of Aramchol was obtained from ethanol. The form was characterized by thermal analysis. The DSC profile showed a first peak at 50.12°C with an onset at 44.87°C (ΔE= -8.45J/g); a second peak at 72.27°C with an onset at 62.58°C (ΔE= 6.28J/g); a third peak at 85.86°C with an onset at 80.06°C (ΔE= -6.20J/g); and a fourth peak at 122.42°C with an onset at 104.82°C (ΔE= -45.78J/g). A continuous weight loss of 25.37% was observed using TGA.

### Example 2 - Solubility of Aramchol Salts:

The Aramchol salts of the present invention were further assessed for their solubility in water. The aqueous solubility was tested at 20°C using the shake-flask method. 5 mg of each salt was weighed. Water was added stepwise until a clear solution was obtained (Table 2, solubility in water). The pH of each solution was measured (Table 2, pH after solubility). The results are summarized in Table 2.

**Table 2.**

| **Base** | **XRPD** | **Solubility in water (mg/ml)** | **pH of solution** |
|---|---|---|---|
| L-Arginine | Amorphous | <11 | n.a. |
| L-Lysine | Amorphous | 10-32 | 8 |
| L-Lysine | Crystalline | 11-35 | 8 |
| Ammonia | Crystalline | <11 | n.a. |
| N-methyl glucamine | Amorphous | 113-1130 | 7 |
| Betaine | Amorphous | <11 | n.a. |
| Betaine | Crystalline | <11 | n.a. |
| Diethylamine | Amorphous | <11 | n.a. |
| Diethylamine | Crystalline | <11 | n.a. |
| Tromethamine | Poorly crystalline | <11 | n.a. |
| Tromethamine | Crystalline | 32-95 | 8 |
| Ethanolamine | Crystalline | <11 | n.a. |
| Diethanolamine | Crystalline | <11 | n.a. |

| | | | |
|---|---|---|---|
| n.a.= not available | | | |

In comparison, Aramchol (free acid) has limited solubility in aqueous media (solubility in buffer at pH 6.0<0.001mg/mL, max solubility of 0.66 mg/ml in FeSSIF, pH=5).

### Example 3:

### Materials and methods:

### X-Ray Powder Diffraction (XRPD)

The X-ray powder diffraction studies were performed using a Bruker AXS D2 PHASER in Bragg-Brentano configuration, equipment #1549. Using a Cu anode at 30kV, 10mA; sample stage standard rotating; monochromatisation by a κβ-filter (0.5% Ni). Slits: fixed divergence slits 1.0mm (=0.61°), primary axial Soller slit 2.5°, secondary axial Soller slit 2.5°. Detector: Linear detector LYNXEYE with receiving slit 5° detector opening. The standard sample holder (0.1 mm cavity in (510) silicon wafer) had a minimal contribution to the background signal.

Measurements conditions: scan range 5-45° 2 , sample rotation 5 rpm, 0.5s/step, 0.010°/step, 3.0mm detector slit; and all measuring condition were logged in the instrument control file. As system suitability, corundum sample (NIST standard) was measured daily.

The software used for data collection is Diffrac.Commander v3.3.35. Data analysis was performed using Diffrac.Eva v 3.0. No background correction or smoothing was applied to the patterns. The contribution of the Cu-Kα₂ was stripped off using the Diffrac.Eva software. Results are summarized in Table 3.

**Table 3.**

| **Base** | **XRPD** |
|---|---|
| L-Arginine | Amorphous |
| L-Lysine | Crystalline material (No salt formation) |
| Ammonia | Crystalline material (No salt formation) |
| N-methylglucamine | Amorphous |
| Betaine | Crystalline material/amorphous |
| Diethylamine | Crystalline material/amorphous (No salt formation) |
| 2-Diethylamino-ethanol | Amorphous |
| Deanol | Crystalline material (No salt formation) |
| Tromethamine | Amorphous/amorphous + additional peak |
| Ethanolamine | Crystalline material (No salt formation) |
| Diethanolamine | Amorphous/amorphous + additional peak (No salt formation) |

### Thermo-Gravimetric Analysis/Differential Scanning Calorimetry (TGA/DSC)

The TGA/DSC were performed using a Mettler Toledo TGA/DSC1 Stare System with a 34-position auto sampler, equipment #1547.

The samples were prepared using aluminum crucibles (40µl; pierced). Typically 5-10mg of each sample was loaded onto a pre-weighed aluminum crucible and was kept at 30°C for 5 minutes, after which it was heated at 10°C/min from 30°C to 300°C. A nitrogen purge was maintained over the sample of 40ml/min. As system suitability check, Indium and Zinc were used as calibration references.

The software used for data collection and evaluation was STARe Software v10.00 build 2480. No corrections were applied to the patterns. Results are summarized in Table 4.

**Table 4.**

| **Base** | **DSC Tₚₑₐₖ (°C)** | **Normalized Integral (J/g)** | **TGA mass loss (%)** |
|---|---|---|---|
| L-Arginine | 50.8 | -17.5 | 8.3 (40-120°C) |
| | 79.2 | -83.5 | 3.7 (200-260°C) |
| | 131.9 | -3.0 | |
| | 238.4 | -80.3 | |
| | 270.4 | -62.2 | |
| | 278.1 | 8.9 | |
| | 283.5 | -12.2 | |
| L-Arginine | 93.5 | -69.0 | 3.3 (40-120°C) |
| | 132.2 | -2.8 | 3.2 (190-250°C) |
| | 230.5 | -21.2 | |
| L-Lysine | 54.8 | -1.5 | 1.1 (40-100°C) |
| | 80.3 | -3.1 | 6.5 (170-250°C) |
| | 117.3 | -45.8 | |
| | 166.4 | -10.9 | |
| | 225.3 | -100.2 | |
| L-Lysine | 92.7 | -4.6 | 3.0 (40-100°C) |
| | 112.4 | -14.6 | 6.1 (160-260°C) |
| | 145.5 | 8.3 | |
| | 166.8 | -14.9 | |
| | 223.9 | -94.9 | |
| Ammonia | 49.4 | -3.5 | 1.2 (40-100°C) |
| | 87.6 | -41.9 | |
| Ammonia | 88.1 | -34.6 | 0.2 (80-100°C) |
| | 151.8 | -11.1 | 0.3 (120-180°C) |
| N-methylglucamine | 49.9 | -25.9 | 8.1 (50-130°C) |
| | 77.2 | -63.8 | |
| | 224.2 | -134.7 | |
| N-methylglucamine | 58.9 | -24.5 | 3.1 (50-130°C) |
| | 79.0 | -28.5 | |
| Betaine | 50.5 | -29.0 | 2.4 (40-100°C) |
| | 65.3 | -13.5 | 2.7 (100-170°C) |
| | 134.4 | -30.2 | 12.9 (200-280°C) |
| | 259.0 | -164.0 | |
| Betaine | 56.5 | 10.6 | 1.9 (40-115°C) |
| | 84.1 | 43.8 | 11.9 (210-280°C) |
| | 261.3 | 159.9 | |
| Diethylamine | 56.7 | -5.4 | 3.2 (40-90°C) |
| | 77.7 | -1.3 | 13.7 (90-220°C) |
| | 106.1 | -51.5 | |
| | 260.6 | -0.9 | |
| Diethylamine | 64.4 | -44.5 | 2.9 (60-110°C) |
| | 99.2 | -7.6 | 2.8 (120-175°C) |
| | 151.1 | -6.6 | |
| | 260.2 | -2.1 | |
| 2-Diethylamino-ethanol | 45.8 | -15.3 | 16.2 (100-210°C) |
| | 108.6 | -28.4 | |
| | 119.6 | -53.3 | |
| | 179.3 | 0.9 | |
| | 198.2 | 2.3 | |
| | 260.7 | -2.1 | |
| Deanol | 87.5 | -12.2 | 20.9 (80-170°C) |
| | 93.9 | -30.7 | |
| | 106.8 | -56.9 | |
| Deanol | 53.4 | -9.1 | 1.0 (60-120°C) |
| | 67.0 | -22.7 | 7.5 (120-220°C) |
| | 138.0 | -28.8 | |
| | 232.6 | 11.3 | |
| Tromethamine | 57.9 | -77.2 | 9.4 (40-110°C) |
| | 205.7 | -130.0 | 8.0 (150-300°C) |
| Tromethamine | 49.0 | -2.3 | 1.4 (100-140°C) |
| | 113.4 | -9.0 | |
| Ethanolamine | 55.0 | -8.5 | 3.6 (50-110°C) |
| | 85.5 | -2.3 | 5.4 (140-220°C) |
| | 105.8 | -13.2 | |
| | 192.7 | -47.7 | |
| Ethanolamine | 103.6 | -53.1 | 0.5 (75-120°C) |
| | 187.7 | -71.1 | 6.2 (125-235°C) |
| Diethanolamine | 49.0 | -14.5 | 1.2 (50-80°C) |
| | 95.3 | -33.0 | 10.8 (85-140°C) |
| | 103.0 | -49.6 | 2.3 (180-240°C) |
| | 202.1 | -28.1 | |
| Diethanolamine | 59.8 | -46.8 | 1.1 (50-90°C) |
| | 77.1 | -26.0 | 5.3 (90-140°C) |
| | 103.2 | -78.5 | 3.0 (175-235°C) |
| | 142.3 | -0.3 | |
| | 205.0 | -25.6 | |

### Dynamic Vapour Sorption (DVS)

The DVS tests were performed using a Surface Measurement System Ltd. DVS-1 No Video, equipment #2126.

The samples was weighed in a glass pan, typically 20-30mg, and equilibrated at 0% relative humidity (RH). After the material had dried, the RH was increased with 10% per step for 1 hour per increment, ending at 95% RH.

The software used for data collection was DVSWin v3.01 No Video. Data analysis was performed using DVS Standard Analysis Suite v6.3.0 (Standard).

Results are summarized in Table 5.

**Table 5.**

| **Base** | **Mass uptake** |
|---|---|
| L-Arginine | 12.5% (stepwise; reversible) |
| L-Lysine | 23.1% (stepwise; reversible) |
| Ammonia | 5.4% (stepwise; reversible) |
| N-methylglucamine | 14.9% (stepwise; reversible) |
| Betaine | 23.0% (stepwise; reversible) |
| Diethylamine | 14.8% (stepwise; reversible) |
| 2-Diethylamino-ethanol | 12.1% (stepwise; reversible) |
| Deanol | 17.3% (stepwise; reversible) |
| Tromethamine | 9.4% (stepwise; reversible) |
| Ethanolamine | 13.2% (stepwise; reversible) |
| Diethanolamine | 6.9% (stepwise; reversible) |

### Polarized Light Microscopy (PLM)

The microscopy studies were performed using an AxioVert 35M, equipped with an AxioCamERc5S, equipment #1612. The microscope was equipped with four lenses, being Zeiss A-Plan 5×/0.12, Zeiss A-Plan 10×/0.25, LD A-Plan 20×/0.30 and Achros TIGMAT 32×/0.40. Data collection and evaluation was performed using Carl Zeiss Zen AxioVision Blue Edition Lite 2011 v1.0.0.0 software.

Results are summarized in Table 6.

**Table 6.**

| **Base** | **PLM** |
|---|---|
| L-Arginine | Rough blocks <20µm |
| L-Arginine | Rounded agglomerated particles <100µm |
| L-Lysine | Small particles <1µm |
| L-Lysine | Agglomerated small particles >100µm |
| Ammonia | Small blocks <20µm |
| Ammonia | Small particles <100µm |
| N-methylglucamine | Blocks <100µm |
| N-methylglucamine | Rounded agglomerated particles >100µm |
| Betaine | Fractured plates >100µm |
| Diethylamine | Fractured plates >100µm |
| 2-Diethylamino-ethanol | Rough blocks >100µm |
| Deanol | Rough blocks >100µm |
| Tromethamine | Agglomerated needles >100µm |
| Ethanolamine | Agglomerated particles >100µm |
| Ethanolamine | Rough blocks >100µm |
| Diethanolamine | Rough blocks >100µm |
| Diethanolamine | Agglomerated small particles >100µm |

### Example 4 - Synthesis and Characterization of Aramchol N-Methyl Glucamine, Tromethamine and Lysine Salts

The synthesis of the N-methylglucamine, tromethamine and lysine salts of Aramchol was accomplished in accordance with General Methods 1 and 2.

General Method 1: An aqueous or alcoholic solution (e.g., methanol, ethanol) of Aramchol and ∼1 molar equivalent of the desired base were heated (e.g., to reflux) until a homogenous solution formed, followed by the addition of an anti-solvent (such as ethyl acetate or acetone) to afford a suspension. The reaction mixture was optionally cooled. The formed salts were isolated by filtration, washed and dried.

Aramchol N-methylglucamine salt was prepared by General Method 1. Aramchol free acid (5.0 g) was mixed with 1.4 g (1 molar equivalent) of N-methylglucamine in water, methanol or ethanol, heated to reflux, followed by adding acetone or ethyl acetate as an anti-solvent, and cooling. A precipitate formed which was isolated and characterized as amorphous Aramchol N-methylglucamine salt. Similar procedures were performed using 1-20 g Aramchol and 1 molar equivalent of N-methylglucamine.

Aramchol lysine salt was prepared by General Method 1. Aramchol free acid (5.0 g) was mixed with 1.0 g (1 molar equivalent) of lysine in methanol or ethanol, heated to reflux, followed by adding acetone or ethyl acetate as an anti-solvent, and cooling. A precipitate formed which was isolated and characterized as amorphous Aramchol lysine salt. Similar procedures were performed using 1-20 g Aramchol and 1 molar equivalent of lysine.

Aramchol tromethamine salt was prepared by General Method 1. Aramchol free acid (5.0 g) was mixed with 0.9 g (1 molar equivalent) of tromethamine in methanol or ethanol, heated to reflux, followed by adding acetone or ethyl acetate as an anti-solvent, and cooling. A precipitate formed which was isolated and characterized as amorphous Aramchol tromethamine salt. Similar procedures were performed using 1-20 g Aramchol and 1 molar equivalent of tromethamine.

General Method 2: An aqueous or alcoholic solution of Aramchol and ∼1 molar equivalent of the desired base were heated (e.g., to reflux) until a homogenous solution formed. The reaction was optionally cooled. The solvent was then removed (e.g., by rotovap under reduced pressure) to afford a solid which was isolated and dried.

Aramchol N-methylglucamine salt was prepared by General Method 2. Aramchol free acid (150.0 g) was mixed with N-methylglucamine (41.7 g) in methanol, and heated to reflux to obtain a homogenous solution. The solution was concentrated on rotovap at 50°C to obtain a solid, which was characterized as amorphous Aramchol N-methylglucamine salt.

Aramchol lysine salt was prepared by General Method 2. Aramchol free acid (50.0 g) was mixed with lysine (10.4 g) in methanol, and heated to reflux to obtain a homogenous solution. The solution was concentrated on rotovap at 50°C to obtain a solid, which was characterized as amorphous Aramchol lysine salt.

Aramchol tromethamine salt was prepared by General Method 2. Aramchol free acid (50.0 g) was mixed with tromethamine (8.6 g) in methanol, and heated to reflux to obtain a homogenous solution. The solution was concentrated on rotovap at 50°C to obtain a solid, which was characterized as amorphous Aramchol tromethamine salt.

### Characterization:

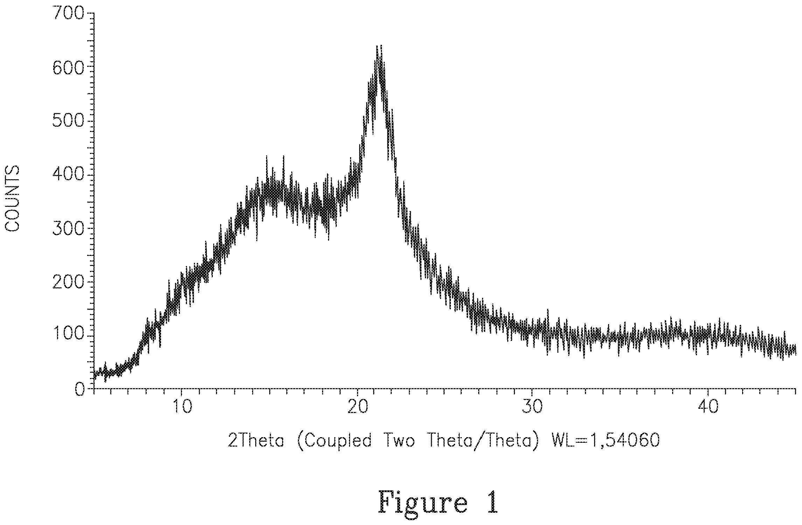

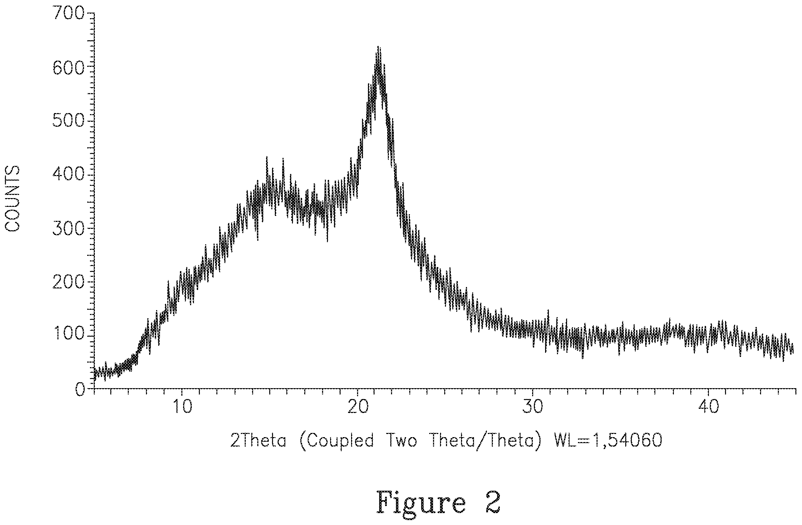

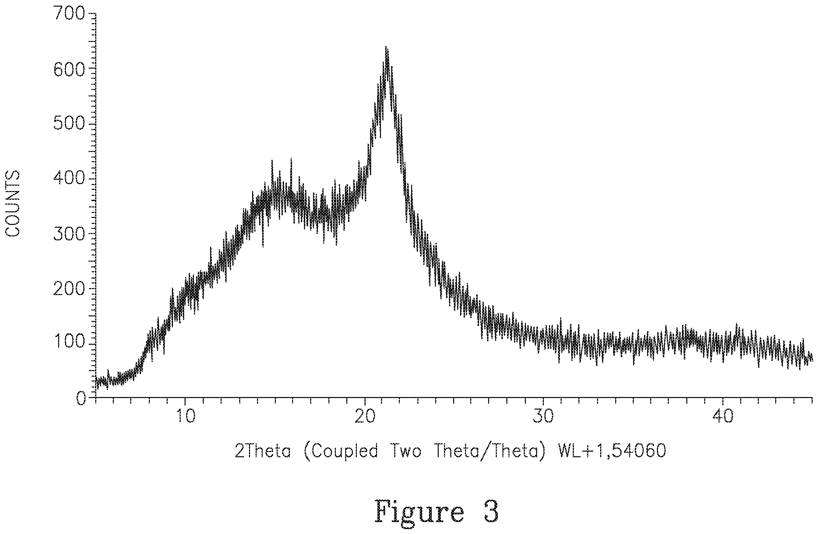

XRPD analyses were performed as described in Example 3, demonstrating that the resulting salts are amorphous. A representative XRPD spectrum of Aramchol N-methylglucamine salt is shown in Figure 1. A representative XRPD spectrum of Aramchol lysine salt is shown in Figure 2. A representative XRPD spectrum of Aramchol tromethamine salt is shown in Figure 3.

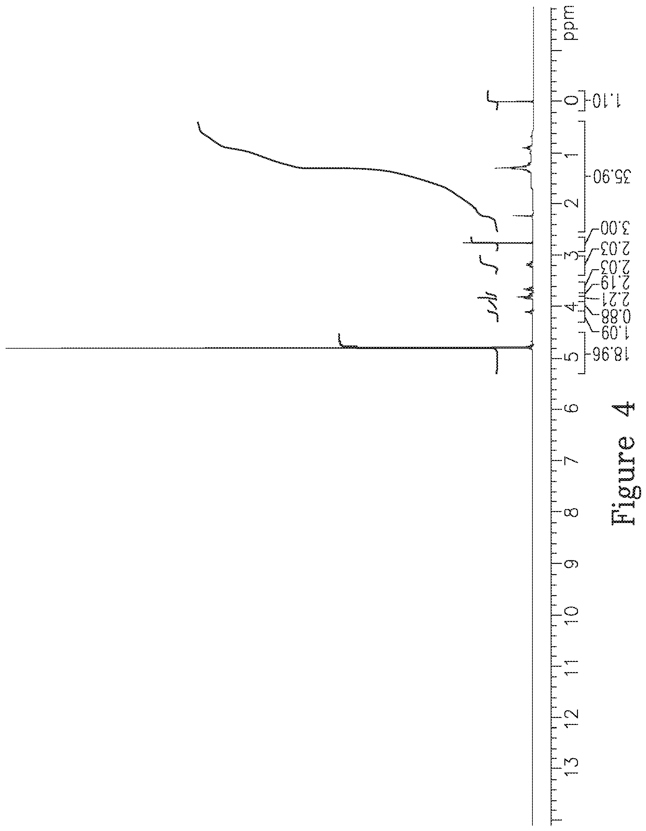

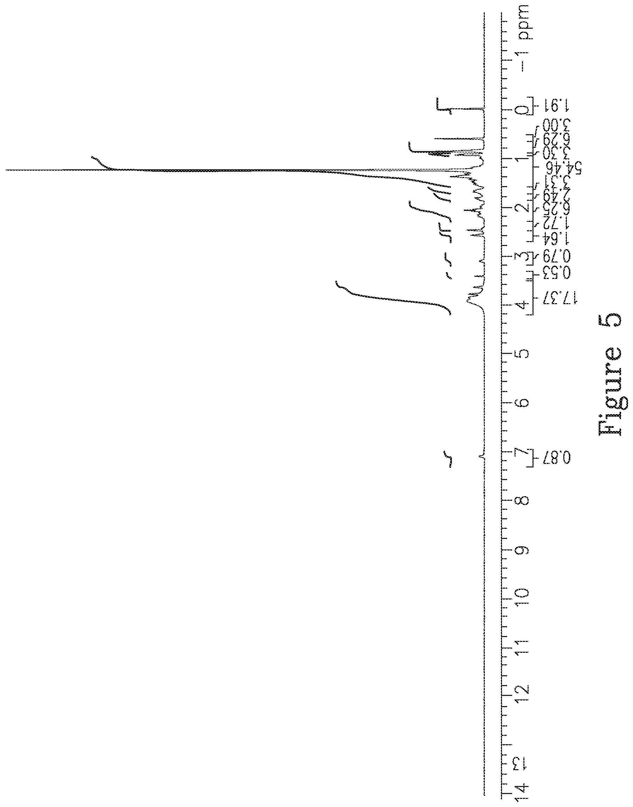

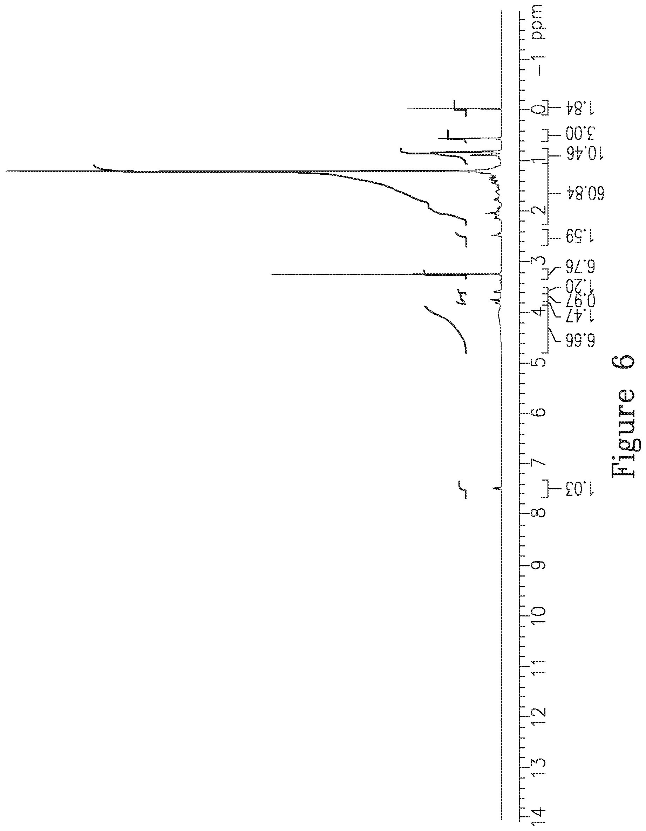

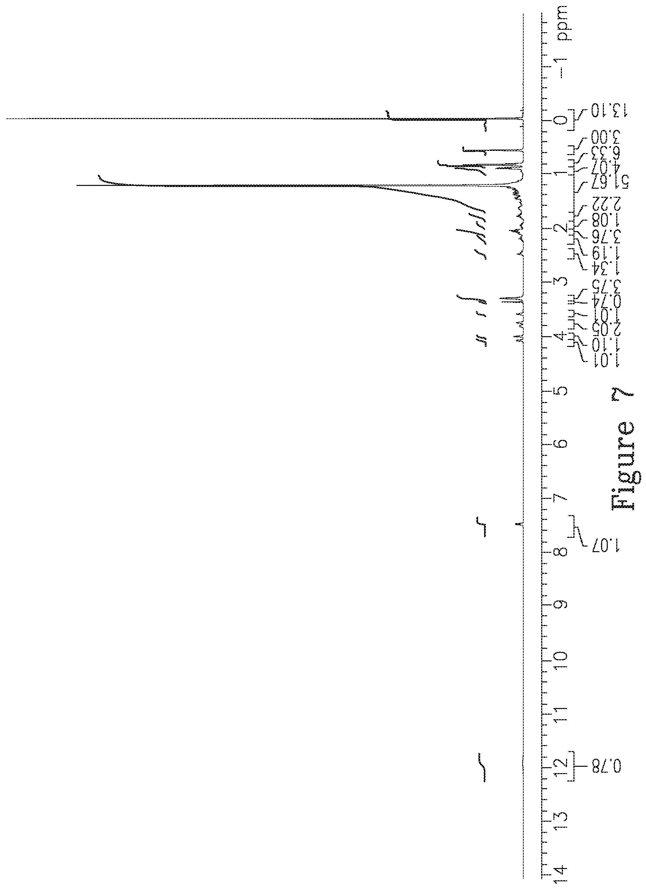

¹H-NMR spectra of the salts were measured, in every case the proton of the carboxylic acid function of Aramchol (located at 12ppm on the NMR spectra) has disappeared, indicating the formation of the salts. A representative ¹H-NMR spectrum of Aramchol N-methylglucamine salt is shown in Figure 4. A representative ¹H-NMR spectrum of Aramchol lysine salt is shown in Figure 5. A representative ¹H-NMR spectrum of Aramchol tromethamine salt is shown in Figure 6. Shown for comparison in Figure 7 is a representative ¹H-NMR spectrum of Aramchol free acid.

### Analytical Measurements:

The following tests were performed on the salts: LC-purity, Karl Fisher (to determine trace amounts of water in a sample) and Loss on drying (LOD) (to measure the mass% which is lost upon heating). The results show similar pattern of water content and % of mass loss among the salts (Table 7).

**Table 7.**

| Entry# | LC-purity (area%) 205 nm | KF (wt%) | LOD (wt%) |
|---|---|---|---|
| Aramchol N-Methylglucamine salt | 98.84 | 1.4 | 1.4 |
| Aramchol Tromethamine salt | 99.05 | 0.9 | 1.1 |
| Aramchol Lysine salt | 96.26 | 1.3 | 1.3 |

### DVS measurements of Aramchol N-Methylglucamine

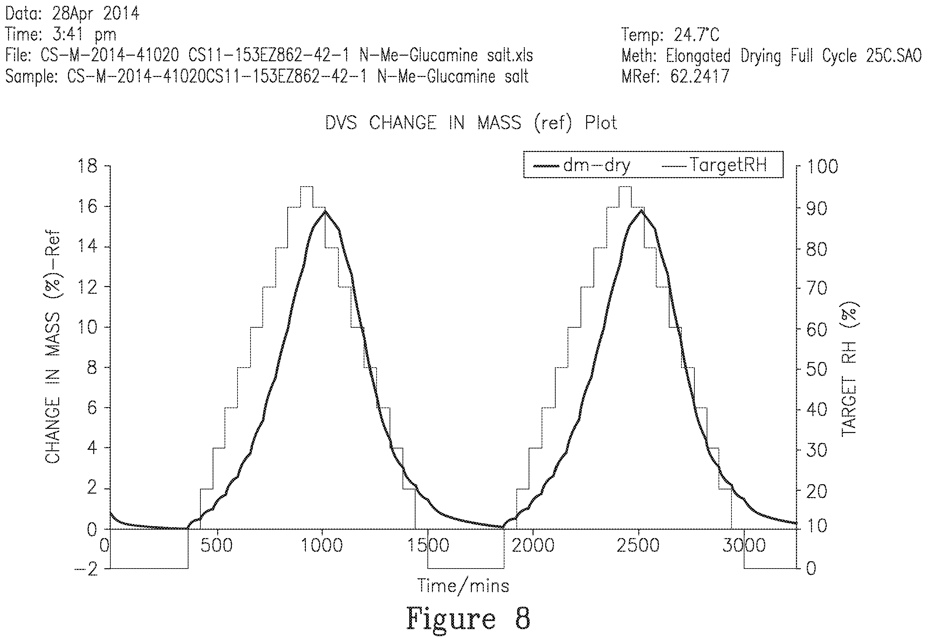

DVS measurements were performed to determine the sorption and desorption behavior of Aramchol N-methylglucamine salt. Sorption was measured by increasing the relative humidity (RH) with 10% per step ending at 95% RH. After completion of sorption cycle, the material was dried. XRPD was performed before and after DVS. DVS showed stepwise sorption in response to change in RH with a total mass uptake of 16%, suggesting that the material is hygroscopic. The sorption was reversible and reproducible. A representative DVS spectrum of the N-methylglucamine salt of Aramchol is depicted in Figure 8. XRPD pattern after DVS showed amorphous material, with different peak shape and intensities (due to different particle size and shape).

### Bulk and tapped density of Aramchol N-Methylglucamine

Measurements of tapped and bulk densities are used to predict the flow properties and compressibility of powders. These two properties are important for manufacture of solid dosage formulations, such as tablets and capsules. Compounds with low values of tapped and bulk densities may be subject to difficulties in tablet compression, and therefore may require additional processing for improving flow properties.

As shown in Table 8, Aramchol (free acid) bulk density is 0.15g/cm³ and tapped density is 0.17g/cm³. Therefore, to improve flow properties a wet granulation process is used prior to tablet compression. For Aramchol N-methylglucamine the measured bulk density is 0.57g/mL and tapped density is 0.66g/mL. The relatively higher values of bulk and tapped density for N-methylglucamine salt (compared to Aramchol free acid), suggest that its improved flow properties may shorten and simplify tablet production procedure by avoiding the additional step of wet granulation.

**Table 8. Tapped and bulk densities**

| **Compound** | **Tapped density** | **Bulk density** |
|---|---|---|
| N methylglucamine salt | 0.66 g/mL | 0.57 g/mL |
| Aramchol (free acid) | 0.17 g/cm³ | 0.15 g/cm³ |

Aramchol (free acid), and the three salts were filled as are, into hard HPMC (Hypromellose Capsule size 00 (CapsCanada, ON, Canada) without taping, fill weight is presented in table 9.

**Table 9: fill weight of one 00 size capsule**

| | |
|---|---|
| Aramchol (free acid) | 0.15 gram |
| Tromethamine salt | 0.31 gram |
| Lysine salt | 0.33 gram |
| N-Me-glucamine salt | 0.30 gram |

The fill volume demonstrate similar tapped volume for three salts

### Example 5. Stability of Aramchol N-Methylglucamine

The N-methylglucamine salt of Aramchol was subjected to accelerated stability according to the following conditions:
a) Exposed to 40°C/75% RH in a closed flask as a solution
b) Exposed to 40°C/75% RH in a closed container in a solid state form
c) Exposed to 40°C/75% RH in an open container in a solid state form

The following parameters were determined at t=0, t=1 week, t=2 weeks: appearance, LC-purity, LC-assay (the assay is calculated against the reference which is the free acid and therefore, the results are less than 100%), water content. Table 10 summarizes the results of stability testing. The appearance and purity remained unchanged under the investigated conditions. Impurity profiling showed neither significant change in impurities present, nor any new significant impurity formed. The calculated assay remained relatively unchanged under the investigational conditions. Water content increased under the investigational conditions and the material seemed hygroscopic. The attraction of water in the solid state form was more prominent for material stored in an open container.

**Table 10. Summarized results of stability**

| | | **as a solution in a closed flask** | | | | **In a solid state form in a closed container** | | | | **In a solid state form in an open container** | | |
|---|---|---|---|---|---|---|---|---|---|---|---|---|
| | | **T=0** | **T= 2** | **T= 1** | | **T=0** | **T= 1** | **T= 2** | | **T=0** | **T= 1** | **T= 2** |
| purity | | 99.5% | 99.5% | 99.5% | | 99.5% | 99.4% | 99.5% | | 99.5% | 99.5% | 99.5% |
| assay | | 74.7% | 74.8% | 75.3% | | 74.7% | 72.8% | 74.4% | | 74.7% | 76.7% | 71.9% |
| water | | not applicable | | | | 1.2% | 1.6% | 2.0% | | 1.2% | 4.3% | 5.7% |

For Aramchol free acid, 6 months stability data have been generated at 40°C / 75% relative humidity and for 12 months at real time 25°C / 60% relative humidity and also at the intermediate conditions of 30°C / 65% relative humidity. Under all conditions and time points there have been no significant changes to any parameters. Thus, comparison of stability of Aramchol free acid and N-methylglucamine demonstrates similar stability profile of both compounds. Moreover, while exposure of the meglumine salt of Aramchol to 40°C/75% RH caused an increase in water content, there was no change to purity values indicating that upon salt formation there is no detrimental change to the stability of Aramchol.

### Example 6. Solubility of N-Methylglucamine, Tromethamine and L-Lysine Aramchol Salts

Aramchol (free acid) has limited solubility in aqueous media (solubility in buffer at pH 6.0<0.001mg/mL, max solubility of 0.66 mg/ml in FeSSIF).

The saturated solubility of N-methylglucamine, Tromethamine and L-Lysine was determined in different buffer solutions and bio-relevant media: HCl buffer pH 1.2, Acetate buffer pH 4.5, Saline pH 5.5, Phosphate buffer pH 6.5, Phosphate buffer pH 7.0, PBS pH 7.4, FaSSIF (pH 6.5), FeSSIF (pH 5.0) and demi-water (pH 7.8, was not adjusted after dissolution). Experiments were performed by slurrying a 5 mL (∼150mg) saturated solution for 30 minutes and 24 hours at 37°C. The exception was water: due to the high solubility ∼1,000 mg was added to 5 mL. All experiments were performed in duplicate. Table 11 demonstrates the solubility of Aramchol salts in selected media.

**Table 11. Overview of the solubility of selected Aramchol salts**

| | | N-Methyl glucamine | Tromethamine | L-Lysine | Aramchol free acid |
|---|---|---|---|---|---|
| pH 1.2 | 30 min | 0 mg/ml | 0.02 mg/ml | 0 mg/ml | n.a. |
| | 24 h | 0 mg/ml | 0.29 mg/ml ± 0.35 | 0 mg/ml | Not soluble |
| pH 4.5 | 30 min | 0 mg/ml | 0 mg/ml | 0 mg/ml | n.a. |
| | 24h | 0 mg/ml | 0 mg/ml | 0 mg/ml | Not soluble |
| pH 5.5 | 30 min | 0.04 mg/ml ± 0.06 | 0.03 mg/ml ± 0.02 | 0.05 mg/ml ± 0.02 | n.a. |
| | 24h | 0.00 mg/ml | 0 mg/ml | 0 mg/ml | Not soluble |
| pH 6.5 | 30 min | Gel | Gel | Gel | n.a. |
| | 24h | Gel | Gel | Gel | <1 µg/mL |
| pH 7.0 | 30 min | 18.85 mg/ml ± 1.88 | 29.39 mg/ml ± 7.45 | 21.16 mg/ml ±3.36 | n.a. |
| | 24h | Gel | Gel | Gel | Not soluble |
| pH 7.4 | 30 min | 31.83 mg/ml ± 2.35 | 22.97 mg/ml ± 3.16 | 32.72 mg/ml ± 1.80 | n.a. |
| | 24h | Gel | Gel | Gel | n.a. |
| FaSSIF | 30 min | Gel | Gel | Gel | 0.05 mg/ml |
| | 24h | Gel | Gel | Gel | 0.13 mg/ml |
| FeSSIF | 30 min | Gel | Gel | Gel | 0.66 mg/ml |
| | 24h | Gel | Gel | Gel | 0.31 mg/ml |
| Demi-Water | 30 min | 156.51 mg/ml ± 24.19 | 45.04 mg/ml ± 1.26 | 49.27 mg/ml ± 0.91 | n.a. |
| | 24h | 109.72 mg/ml ± 8.61 | Gel | Gel | Not soluble |

| | | | | | |
|---|---|---|---|---|---|
| Data arithmetic mean ± standard deviation n.a. not available | | | | | |

The results show that solubility of Aramchol salts is pH dependent: at acidic pH (pH 1.2-6.5) it is poorly soluble, with solubility increasing at pH 7 and above. At pH 7, 7.4 similar solubilities are demonstrated for all three salts. However, surprisingly, a relatively large increase in solubility (5 fold) is demonstrated for N-methylglucamine salt upon increase of pH from 7.4 (PBS) to pH 7.8 (demi-water), compared to the two other salts.
Overall, comparison of solubility between Aramchol (free acid) and salts demonstrates higher solubility for Aramchol salts at physiological relevant pH (30,000 fold increase in concentration at pH 7.4).

### Example 7. In vivo permeability experiments in cannulated rats

An *in vivo* permeability study of Aramchol salts was performed in male Wistar rats cannulated in the jugular vein and in the jejunum. Intestinal cannulation was performed in order to bypass protonation of Aramchol salts in acidic gastric pH. Aramchol salts solubilized in PBS (30mg/mL) were administered to rats intestine (jejunum) in a dose of 100mg/kg (based on free acid), via a cannula inserted into the proximal side of the jejunum. A suspension of Aramchol free acid (in PBS, 30mg/mL) was administered via the same route and was used as control. Blood samples were withdrawn via a cannula inserted into jugular vein at pre-determined time points (pre-dose, 1hr, 2hr, 4hr, 8hr, 12hr, 24hr post dose). Plasma concentrations of Aramchol (free acid) were measured using a liquid chromatography-tandem mass spectrometry (LC-MS-MS) method by Analyst Bioanalytical Laboratories, Israel. All PK parameters were calculated using non-compartmental analysis. Only those plasma concentrations equal to or greater than the lower limit of quantitation (LOQ) (48.66 ng/mL) were used in the analysis. Plasma concentrations < LOQ that occurred from pre-dose to the first concentration ≥ LOQ were treated as 0. Actual sampling times were used for all pharmacokinetic analyses. The following PK parameters were calculated: maximum plasma concentration (Cₘₐₓ), time to Cₘₐₓ (Tₘₐₓ), area under the plasma concentration-time curve from time of administration until the last plasma concentration (AUC₀₋ₜ), AUC/dose, elimination half-life (t½). Cₘₐₓ and Tₘₐₓ were taken directly from the data. Area under the curve from zero to the final sample with a concentration ≥ LOQ. AUC₀₋ₜ was calculated using the linear trapezoidal method.

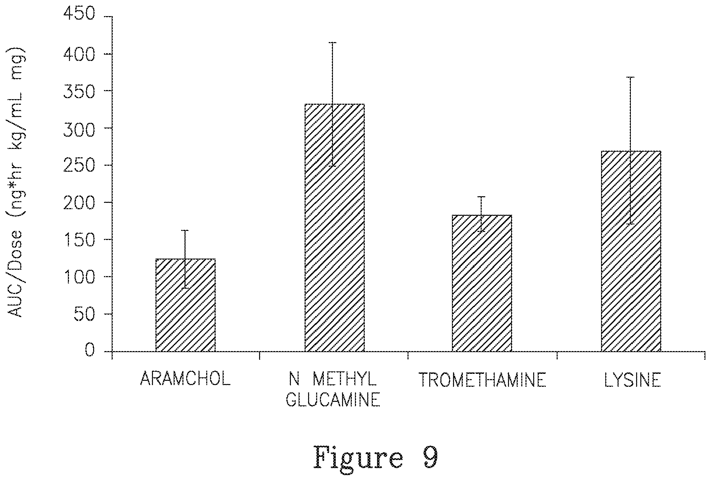

As shown in Table 12, the mean ± standard error Cₘₐₓ and AUC/dose of Aramchol (free acid) were lower compared to the three salts N-methylglucamine, lysine and tromethamine. A substantial increase in both AUC/dose and Cₘₐₓ was observed for N-methylglucamine salt, compared to Aramchol free acid (Figure 9). Averaged across the 2 parameters, the increase was 2.6 fold and 3.6 fold for AUC/dose and Cₘₐₓ, respectively.

Taken together the data show increased systemic exposure for all Aramchol salts, compared to free acid form, supporting the role of aqueous solubility in absorption of Aramchol.

**Table 12. Summary of PK parameters for Aramchol (free acid) after intrajejunal administration of Aramchol and Aramchol salts**

| Parameter | Aramchol (free acid) | N-Methylglucamine salt | Lysine salt | Tromethamine salt |
|---|---|---|---|---|
| Cₘₐₓ (ng/mL) | 1362.3 ± 359.1 (5) | 5012.1 ± 1879.9 (5) | 7294.2 ± 5463.0 (5) | 2254.9 ± 208.3 (4) |
| Tₘₐₓ (hr) | 4.0 (5) | 4.0 (5) [2-4] | 2.0 (5) [2-4] | 2.0 (4) [2-4] |
| AUC₀₋ₜ (hr x ng/mL) | 12129.7 ± 3626.2 (5) | 33625.2 ± 9567.7 (5) | 26460.3 ± 9415.5 (5) | 18583.9± 2283.8 (4) |
| AUC/dose (hr x ng x kg /mL x mg) | 124.2 ± 38.9 (5) | 331.7 ± 82.5 (5) | 270.0 ± 99.0 (5) | 184.7 ± 22.7 (4) |
| t_{½} (hr) | 4.5 (1) | 5.2 ± 1.0 (5) | 5.2 ± 1.0 (5) | 6.5 ± 2.4 (4) |

| | | | | |
|---|---|---|---|---|
| Arithmetic mean ± standard error (N) except for Tₘₐₓ for which the median (N) [Range] is reported. N: number of animals in each group. | | | | |

### Conclusions

About 30 pharmaceutically acceptable bases were screened in an effort to prepare Aramchol salts. Of them, amine-based salts were found to be suitable and in particular three salts of Aramchol have been selected as preferred salts. As demonstrated herein, the N-methylglucamine, lysine and tromethamine salts of Aramchol have been prepared and have been shown to possess advantageous properties. Several unexpected findings related to Aramchol salts in general, and the three preferred salts in particular, are summarized hereinbelow.
1) The selection of a suitable base for formation of pharmaceutically suitable Aramchol salts is not trivial. There is no clear correlation of the base molecular weight, pKa, presence of polar groups, or steric factors on salt formation.
2) Substantial solubility differences across a narrow pH range (7.0-7.8) were also unexpected. For example the three tested salts show similar solubility in pH 7 and 7.4. However, solubility of N-methylglucamine in demi-water (pH 7.8) is 5 fold higher than in pH 7.4, while for the other two salts the difference is relatively low.
3) Prediction of solution stability is unexpected. For example, the N-methylglucamine salt shows relatively higher stability in solution as compared with the other two salts (Table 11). For example, at pH=7.8 (demi-water), both the tromethamine salt and lysine salt solutions turned into gets after 24 hours, while the N-methylglucamine salt remained as a solution.

In addition, there are several advantageous properties of the tested Aramchol salts as compared with Aramchol free acid:
*In vitro* solubility of Aramchol salts is correlated to their *in vivo* absorption: The increased solubility of the three salts, compared to Aramchol free acid in physiological medium (pH buffer 7-7.8) results in increased exposure (measured by Cₘₐₓ and AUC). Moreover, higher exposure of N-methylglucamine compared to lysine and tromethamine salts may be correlated to its increased stability in solution.

Finally, the relatively higher values of bulk and tapped density for N-methylglucamine salt (compared to Aramchol free acid) suggest that its improved flow properties may facilitate simpler tablet production procedure by avoiding the additional step of wet granulation or other steps designed to overcome to compresability problem of low density powders and the steps needed to enable hard capsules filling.

## Claims

1. A salt of 3β-arachidylamido-7α,12α-dihydroxy-5β-cholan-24-oic acid with an amine.

2. The salt according to claim 1, wherein the amine is selected from the group consisting of ammonia, a primary amine, a secondary amine, a tertiary amine, a quaternary ammonium compound, an amino alcohol, an amino sugar and an amino acid; preferably wherein the amine is selected from the group consisting of an amino alcohol, an amino sugar and an amino acid.

3. The salt according to claim 1 selected from the group consisting of ammonium, benzathine, trimethylglycine (betaine), ethanolamine, diethanolamine, diethylamine, arginine, lysine, choline, deanol, 2-diethylaminoethanol, N-methylglucamine (meglumine), N-ethylglucamine (eglumine) and tromethamine salts.

4. The salt of according to claim 1, which is selected from the group consisting of:
3β-arachidylamido-7α,12α-dihydroxy-5β-cholan-24-oic acid lysine salt;
3β-arachidylamido-7α,12α-dihydroxy-5β-cholan-24-oic acid tromethamine salt; and
3β-arachidylamido-7α,12α-dihydroxy-5β-cholan-24-oic acid N-methylglucamine salt.

5. The salt according to any one of claims 1 to 4, which is in a crystalline form.

6. The salt according to any one of claims 1 to 4, which is in an amorphous form.

7. A method of preparing the salt according to any one of claims 1 to 6, the method comprising the steps of:
(a) mixing 3β-arachidylamido-7α,12α-dihydroxy-5β-cholan-24-oic acid with an amine in the presence of a solvent;
(b) optionally heating the mixture to a temperature at or below the solvent boiling point;
(c) optionally cooling the mixture; and
(d) isolating the thus obtained amine salt of 3β-arachidylamido-7α,12α-dihydroxy-5β-cholan-24-oic acid.

8. A method of preparing the salt according to any one of claims 1 to 6, the method comprising the steps of:
(a) mixing 3β-arachidylamido-7α,12α-dihydroxy-5β-cholan-24-oic acid with an amine in the presence of a solvent;
(b) optionally heating the mixture to a temperature at or below the solvent boiling point;
(c) adding an anti-solvent;
(c) optionally cooling the mixture; and
(d) isolating the thus obtained amine salt of 3β-arachidylamido-7α,12α-dihydroxy-5β-cholan-24-oic acid.

9. The method according to claim 7 or 8, wherein the solvent is selected from water, an alcohol and ethyl acetate.

10. The method according to claim 8, wherein the anti-solvent is acetone or ethyl acetate.

11. The method according to claim 7 or 8, wherein the amine is selected from the group consisting of ammonia, a primary amine, a secondary amine, a tertiary amine, a quaternary ammonium compound, an amino alcohol, an amino sugar and an amino acid.

12. A pharmaceutical composition comprising a therapeutically effective amount of a salt according to any one of claims 1 to 6 and optionally at least one pharmaceutically acceptable carrier, diluent, vehicle or excipient,
preferably wherein the composition is in a form selected from the group consisting of tablets, pills, capsules, pellets, granules, powders, lozenges, sachets, cachets, patches, elixirs, suspensions, dispersions, emulsions, solutions, syrups, aerosols, ointments, soft and hard gelatin capsules, suppositories, sterile injectable solutions, and sterile packaged powders,
more preferably wherein the composition is suitable for administering via an oral, transdermal or topical route.

13. The pharmaceutical composition of claim 12 for use in:
reducing cholesterol levels in the blood or treating fatty liver; or
treating Non Alcoholic SteatoHepatitis (NASH); or
dissolving cholesterol gallstones in bile and for preventing formation of such gallstones; or
treating arteriosclerosis; or
treating a disease or disorder associated with altered glucose metabolism; or
treating, preventing, or inhibiting progression of a brain disease **characterized by** amyloid plaque deposits.

14. The pharmaceutical composition for use of claim 13, wherein the disease or disorder associated with altered glucose metabolism is selected from the group consisting of hyperglycemia, diabetes, insulin resistance and obesity.

15. The pharmaceutical composition for use of claim 13, wherein the brain disease **characterized by** amyloid plaque deposits is Alzheimer's disease.

## Patentansprüche

1. Salz einer 3β-Arachidylamido-7α,12α-dihydroxy-5β-cholan-24-säure mit einem Amin.

2. Salz nach Anspruch 1, wobei das Amin von der Gruppe bestehend aus Ammoniak, einem Primäramin, einem Sekundäramin, einem Tertiäramin, einer quaternären Ammoniumverbindung, einem Aminoalkohol, einem Aminozucker und einer Aminosäure ausgewählt ist; wobei das Amin bevorzugt von der Gruppe bestehend aus einem Aminoalkohol, einem Aminozucker und einer Aminosäure ausgewählt ist.

3. Salz nach Anspruch 1, das von der Gruppe bestehend aus Ammonium, Benzathin, Trimethylglycin (Betain), Ethanolamin, Diethanolamin, Diethylamin, Arginin, Lysin, Cholin, Deanol, 2-Diethylaminoethanol, N-Methylglucamin (Meglumin), N-Ethylglucamin (Eglumin) und Tromethaminsalzen ausgewählt ist.

4. Salz nach Anspruch 1, das von der folgenden Gruppe ausgewählt wird:
3β-Arachidylamido-7α,12α-dihydroxy-5β-cholan-24-säure-Lysinsalz;
3β-Arachidylamido-7α,12α-dihydroxy-5β-cholan-24-säure-Tromethaminsalz; und
3β-Arachidylamido-7α,12α-dihydroxy-5βB-cholan-24-säure-N-Methylglucaminsalz.

5. Salz nach einem der Ansprüche 1 bis 4, das in Kristallform vorliegt.

6. Salz nach einem der Ansprüche 1 bis 4, das in einer amorphen Form vorliegt.

7. Verfahren zur Herstellung des Salzes nach einem der Ansprüche 1 bis 6, wobei das Verfahren die folgenden Schritte umfasst:
(a) Versetzen von 3β-Arachidylamido-7α,12α-dihydroxy-5β-cholan-24-säure mit einem Amin in Gegenwart eines Lösungsmittels;
(b) Wahlweises Erhitzen des Gemischs auf eine Temperatur, die beim Kochpunkt des Lösungsmittels oder darunter liegt;
(c) Wahlweises Abkühlen des Gemischs; und
(d) Isolieren des so erhaltenen Aminsalzes von 3β-Arachidylamido-7α,12α-dihydroxy-5β-cholan-24-säure.

8. Verfahren zur Herstellung des Salzes nach einem der Ansprüche 1 bis 6, wobei das Verfahren die folgenden Schritte umfasst:
(a) Versetzen von 3β-Arachidylamido-7a,12a-dihydroxy-5β-cholan-24-säure mit einem Amin in Gegenwart eines Lösungsmittels;
(b) Wahlweises Erhitzen des Gemischs auf eine Temperatur, die beim Kochpunkt des Lösungsmittels oder darunter liegt;
(c) Zusetzen eines Antilösungsmittels;
(d) Isolieren des so erhaltenen Aminsalzes von 3β-Arachidylamido-7α,12α-dihydroxy-5β-cholan-24-säure.

9. Verfahren nach Anspruch 7 oder 8, wobei das Lösungsmittel aus Wasser, einem Alkohol und Ethylacetat ausgewählt ist.

10. Verfahren nach Anspruch 8, wobei das Antilösungsmittel Aceton oder Ethylacetat ist.

11. Verfahren nach Anspruch 7 oder 8, wobei das Amin von der Gruppe bestehend aus Ammoniak, einem Primäramin, einem Sekundäramin, einem Tertiäramin, einer quaternären Ammoniumverbindung, einem Aminoalkohol, einem Aminozucker und einer Aminosäure ausgewählt wird.

12. Pharmazeutische Zusammensetzung, umfassend eine therapeutisch wirksame Menge eines Salzes nach einem der Ansprüche 1 bis 6 und wahlweise mindestens einen pharmazeutisch annehmbaren Träger, Verdünner, Trägerstoff oder Hilfsstoff,
wobei die Zusammensetzung bevorzugt in einer Form vorliegt, die von der folgenden Gruppe ausgewählt ist: Tabletten, Pillen, Kapseln, Pellets, Körnchen, Pulvern, Lutschbonbons, Beuteln, Kapseln aus Stärkemasse, Patches, Elixieren, Suspensionen, Dispersionen, Emulsionen, Lösungen, Sirupen, Aerosolen, Salben, weichen und harten Gelatinekapseln, Zäpfchen, steril-injizierbaren Lösungen und steril verpackten Pulvern,
noch bevorzugter, wobei die Zusammensetzung dazu geeignet ist, über einen oralen, transdermalen oder topischen Verabreichungsweg verabreicht zu werden.

13. Pharmazeutische Zusammensetzung nach Anspruch 12 zur Verwendung beim:
Reduzieren des Cholesterinspiegels im Blut oder zum Behandeln einer Fettleber; oder
Behandeln von Nichtalkoholischer Steatohepatitis (NASH); oder
Auflösen von Cholesteringallensteinen in Gallensaft und zum Vorbeugen gegen die Bildung solcher Gallensteine; oder
Behandeln von Arteriosklerose; oder
Behandeln einer Krankheit oder Störung, die mit einem veränderten Glukosestoffwechsel in Verbindung gebracht wird; oder
Behandeln, Vorbeugen oder Hemmen des Fortschreitens einer Erkrankung des Gehirns, die durch die Ablagerung von Amyloid-Plaques gekennzeichnet ist.

14. Pharmazeutische Zusammensetzung zur Verwendung nach Anspruch 13, wobei die Krankheit oder Störung mit einem veränderten Glukosestoffwechsel in Zusammenhang gebracht wird und von der Gruppe bestehend aus Hyperglykämie, Diabetes, Insulinresistenz und Adipositas ausgewählt ist.

15. Pharmazeutische Zusammensetzung zur Verwendung nach Anspruch 13, wobei die Erkrankung des Gehirns, die durch Ablagerungen von Amyloid-Plaques gekennzeichnet ist, Morbus Alzheimer ist.

## Revendications

1. Sel de l'acide β-arachidylamido-7α,12α-dihydroxy-5β-cholan-24-oïque avec une amine.

2. Sel selon la revendication 1, dans lequel l'amine est choisie dans le groupe constitué par l'ammoniac, une amine primaire, une amine secondaire, une amine tertiaire, un composé d'ammonium quaternaire, un aminoalcool, un sucre aminé et un acide aminé ; de préférence dans lequel l'amine est choisie dans le groupe constitué par un aminoalcool, un sucre aminé et un acide aminé.

3. Sel selon la revendication 1, choisi dans le groupe constitué d'ammonium, benzathine, triméthylglycine (bétaïne), éthanolamine, diéthanolamine, diéthylamine, arginine, lysine, choline, déanol, 2-diéthylaminoéthanol, N-méthylglucamine (méglumine), N-éthylglucamine (méglumine) et des sels de trométhamine.

4. Sel selon la revendication 1, lequel est choisi dans le groupe constitué de :
Sel lysine d'acide 3β-arachidylamido-7α,12α-dihydroxy-5β-cholan-24-oïque ;
Sel trométhamine d'acide 3β-arachidylamido-7α,12α-dihydroxy-5β-cholan-24-oïque ; et
Sel N-méthylglucamine d'acide 3β-arachidylamido-7α,12α-dihydroxy-5β-cholan-24-oïque.

5. Sel selon l'une quelconque des revendications 1 à 4, qui est sous une forme cristalline.

6. Sel selon l'une quelconque des revendications 1 à 4, qui est sous une forme amorphe.

7. Procédé de préparation du sel selon l'une quelconque des revendications 1 à 6, le procédé comprenant les étapes consistant à :
(a) mélanger l'acide 3β-arachidylamido-7α,12α-dihydroxy-5β-cholan-24-oïque avec une amine en présence d'un solvant ;
(b) chauffer éventuellement le mélange à une température égale ou inférieure au point d'ébullition du solvant ;
(c) éventuellement refroidir le mélange ; et
(d) isoler le sel amine ainsi obtenu de l'acide 3β-arachidylamido-7α,12α-dihydroxy-5β-cholan-24-oïque.

8. Procédé de préparation du sel selon l'une quelconque des revendications 1 à 6, le procédé comprenant les étapes consistant à :
(a) mélanger l'acide 3β-arachidylamido-7α,12α-dihydroxy-5β-cholan-24-oïque avec une amine en présence d'un solvant ;
(b) éventuellement chauffer le mélange à une température égale ou inférieure à la température du point d'ébullition du solvant ;
(c) ajouter un anti-solvant ;
(c) éventuellement refroidir le mélange ; et
(d) isoler le sel amine ainsi obtenu de l'acide 3β-arachidylamido-7α,12α-dihydroxy-5β-cholan-24-oïque.

9. Procédé selon la revendication 7 ou 8, dans lequel le solvant est choisi parmi de l'eau, un alcool et de l'acétate d'éthyle.

10. Procédé selon la revendication 8, dans lequel l'anti-solvant est l'acétone ou l'acétate d'éthyle.

11. Procédé selon la revendication 7 ou 8, dans lequel l'amine est choisie dans le groupe comprenant l'ammoniac, une amine primaire, une amine secondaire, une amine tertiaire, un composé ammonium quaternaire, un aminoalcool, un sucre aminé et un acide aminé.

12. Composition pharmaceutique comprenant une quantité thérapeutiquement efficace d'un sel selon l'une quelconque des revendications 1 à 6 et éventuellement au moins un support, un diluant, un véhicule ou un excipient acceptable d'un point de vue pharmaceutique,
de préférence dans laquelle la composition est sous une forme choisie dans le groupe comprenant des comprimés, pilules, gélules, pellets, granulés, poudres, pastilles, sachets, cachets, timbres, élixirs, suspensions, dispersions, émulsions, solutions, sirops, aérosols, pommades, gélules molles et dures, suppositoires, solutions injectables stériles et poudres emballées stériles,
de manière davantage préférée, dans laquelle la composition est appropriée pour une administration par voie orale, transdermique ou topique.

13. Composition pharmaceutique selon la revendications 12, destinée à être utilisée pour :
réduire le taux de cholestérol dans le sang ou traiter un foie gras ; ou
traiter une stéatohépatite alcoolique (ASH) ; ou
dissoudre des calculs biliaires de cholestérol dans la bile et prévenir la formation de tels calculs biliaires ; ou
traiter l'artériosclérose ; ou
traiter une maladie ou un trouble associé à une altération du métabolisme du glucose ; ou
traiter, prévenir ou inhiber la progression d'une maladie cérébrale **caractérisée par** des dépôts de plaque amyloïde.

14. Composition pharmaceutique à utiliser selon la revendication 13, dans laquelle la maladie ou le trouble associé à un métabolisme altéré du glucose est choisi dans le groupe comprenant l'hyperglycémie, le diabète, la résistance à l'insuline et l'obésité.

15. Composition pharmaceutique à utiliser selon la revendication 13, dans laquelle la maladie cérébrale **caractérisée par** des dépôts de plaque amyloïde est la maladie d'Alzheimer.
